(19) Europäisches Patentamt

European Patent Office

Office européen des brevets

(11) **EP 0 904 128 B1**

(12) **EUROPEAN PATENT SPECIFICATION**

(45) Date of publication and mention
of the grant of the patent:
**14.01.2004 Bulletin 2004/03**

(51) Int Cl.⁷: **A61N 1/30**, A61K 9/70

(21) Application number: **97923130.5**

(86) International application number:
**PCT/FI1997/000319**

(22) Date of filing: **27.05.1997**

(87) International publication number:
**WO 1997/047353 (18.12.1997 Gazette 1997/54)**

(54) **COMPOSITION AND SYSTEM FOR IONTOPHORETIC TRANSDERMAL DELIVERY OF DRUGS**

ZUSAMMENSETZUNG UND SYSTEM ZUR IONTOPHORETISCHEN TRANSDERMALEN ABGABE VON MEDIKAMENTEN

COMPOSITION ET SYSTEME D'ADMINISTRATION TRANSDERMIQUE ET IONTOPHORETIQUE DE MEDICAMENTS

(84) Designated Contracting States:
**AT BE CH DE DK ES FI FR GB GR IE IT LI LU MC NL PT SE**

(30) Priority: **13.06.1996 FI 962461**

(43) Date of publication of application:
**31.03.1999 Bulletin 1999/13**

(73) Proprietor: **Novagent Oy**
**02700 Kauniainen (FI)**

(72) Inventors:
• **JÄRNSTRÖM, Risto**
**FIN-00180 Helsinki (FI)**
• **HIRVONEN, Jouni**
**FIN-01390 Vantaa (FI)**

(74) Representative: **Öhman, Ann-Marie**
**Turun Patenttitoimisto Oy,**
**P.O. Box 99**
**20521 Turku (FI)**

(56) References cited:
WO-A-92/15365  US-A- 1 967 927
US-A- 4 585 652  US-A- 5 098 417

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

This invention relates to a composition and a system for controlled release of a drug in iontophoretic transdermal administration.

BACKGROUND OF THE INVENTION

The publications and other materials used herein to illuminate the background of the invention, and in particular, cases to provide additional details respecting the practice, are incorporated by reference.

Transdermal delivery is a feasible alternative route of drug administration for many drugs. Drugs whose daily dose is 20-30 mg or less, are potential candidates for transdermal drug delivery (Guy and Hadgraft 1987, Guy and Hadgraft 1989).

Transdermal administration of therapeutically active agents is usually accomplished by incorporating the drug into a transdermal delivery device which is able to control the delivery rate of the drug. According to one alternative the transdermal device comprises a backing layer, an adhesive layer and a matrix layer preferably made of a polymer material in which the drug is dispersed. The rate of which the drug is released from the device is here controlled by the polymer matrix. Another kind of trensdermal device is the reservoir system comprising a) a drug impermeable backing layer, b) an adhesive layer, c) a drug permeable membrane sealed to one side of the backing layer as to define a drug reservoir compartment there between, and d) a drug or composition thereof within said drug reservoir. In this case the drug in the reservoir is usually in liquid or gel form. The drug permeable membrane controls the rate at which the drug is delivered to the skin.

US 4,692,462 describes a composition and method for controlled transdermal delivery based on the use of a ion exchange resin loaded with the drug to be administered. This drug-loaded ion exchange resin is, together with a salt that is able to release the drug from the ion exchange resin, mixed with a gel-forming vehicle and incorporated in a device having a cavity, an adhesive layer and a backing layer.

Iontophoresis or iontophoretic therapy is the delivery into mammal tissue of a drug by the use of an electric current. For reviews of methods, devices and drugs suitable for iontophoretic delivery, see e.g. Journal of Controlled release Vol. 7, 1988, pp. 1-24; Drug Design and Delivery Vol. 4, 1989, pp. 1-12 and Journal of Pharmaceutical Sciences Vol. 78, 1989, No. 5, pp. 376-383.

SUMMARY OF THE INVENTION

The object of this invention is to provide means to increase and control the transdermal delivery of a drug from a drug composition.

The invention thus concerns a pharmaceutical composition for the controlled iontophoretic transdermal delivery of a drug comprising a combination of a drug and an electrically conductive carrier, wherein the administration of the drug to the patient is intended to be enhanced by leading an electrical current from said composition to the patient's skin. According to the invention, the electrically conductive carrier is a textil fiber with a ion exchanger group grafted thereto.

Furthermore, this invention concerns a system for the iontophoretic transdermal delivery of a drug to a patient, wherein said system comprises (a) a first device comprising the drug to be administered; (b) a second device, wherein said devices are intended to be attached to the patient's skin; (c) a source of direct current; and (d) means for connecting said current source to said first device and to said second device. The system is characterized in that said first device is a pharmaceutical composition according to this invention.

BRIEF DESCRIPTION OF THE DRAWINGS

Figure 1 shows a iontophoretic delivery system according to this invention,

Figure 2 shows the principle of iontophoretic drug delivery,

Figure 3 shows the apparatus used in the test of the drug delivery,

Figure 4 shows the *in vitro* release of sodium salicylate from an anionic ion exchange textil fiber in PBS solution versus time (143 mM, $\mu$ = 0.15; Mean $\pm$ SE, N = 7),

Figure 5 shows the *in vitro* release of sodium salicylate from an anionic ion exchange textil fiber in phosphate buffer (pH 7.4) versus time (6 mM, $\mu$ = 0.2; Mean $\pm$ SE, N = 8),

Figure 6 shows the in vitro release of sodium salicylate from an anionic ion exchange textil fiber in phosphate buffer (pH 7.4) through skin versus time (6 mM, $\mu$ = 0.2; Mean $\pm$ SE, N = 8),

Figure 7 shows the *in vitro* release of tacrine from a cationic ion-exchange fiber at pH 7.4. Average $\pm$ SD, N = 4,

Figure 8 shows tacrine permeation across human skin from 5 % solution *in vitro.* Average $\pm$ SD, N = 5,

Figure 9 shows tacrine permeation from a cationic ion-exchange fiber across human skin *in vitro,* average $\pm$ SD, N = 7,

Figure 10 shows the in vitro permeation of tacrine across human skin from 5 % solution. Mean $\pm$ SD, N = 6. Constant current iontophoresis (0.5 mA/cm$^2$) was on for 12 h, and

Figure 11 shows the in vitro permeation of tacrine across human skin from cationic ion-exchange fiber. Mean $\pm$ SD, N = 6. Constant current iontophoresis (0.5 mA/cm$^2$) was on for 12 h.

## DETAILED DESCRIPTION OF THE INVENTION

Suitable fibers for use in this invention are any pharmaceutically acceptable textile fibers of native or synthetic origin. Examples of such fibers are wool, cotton, flax fibers and fibers of cellulose or its derivatives, polyethylene, polypropylene, polystyrene, polyamide fibers and carbon fibers.

Said fibers can be grafted with positive functional groups such as -N$^+$(CH$_3$)$_3$ (trimethylammonium), -NH$^+$(CH$_3$)$_2$ (dimethylammonium), or the like to give fibers having the ability to bind and release negative groups (anions), i.e. to give anionic exchangers. If the fibers are grafted with negative functional groups such as -COOH (carboxylic) or -SO$^-_3$ (sulphonic), cationic exhangers are obtained.

The choice of the functional group depends on the properties of the drug to be administered, the desired loading and the desirable administration rate. The amount of functional groups grafted onto the fiber affects the loading capacity of the fiber.

The use of fibers grafted with suitable ion exchanger group possesses a considerable advantage over the use of ion exhange resins with respect to the kinetic properties. The loading and delivery of the active substance is much faster from a fiber than from a resin because the contact between the functional ion exchanger group is much better if said functional group is attached to a fiber than to a resin. The disadvantages of the resin are due to 1) great dimensions of the resin sphere compared to the cross section of a fiber, and 2) the cross-linking of the polymer in the resin restricts the motility of the functional group.

Suitable drugs to be administered by the anionic exchanger are typically acids having a -COOH group such as acetylsalicylic acid, indomethacin, furosemide, acetaminophen, levodopa, prostaglandins and the like. Drugs that can be administered by a cationic exchanger are basic compounds such as clonidine, dopamine, chlorpromazine, benzodiazepines, beta-blockers such as propranolol etc., selegiline and nicotine.

Examples of suitable salts for the release of the drug from the ion exchanger are sodium chloride, sodium phosphate (mono- or dibasic), zinc sulfate, magnesium chloride, calcium chloride, potassium chloride, sodium sulfate, magnesium acetate and sodium citrate.

A particularly preferred compound is the anticholinesterase inhibitor tacrine (9-amino-1,2,3,4-tetrahydroacridine) and its pharmaceutically acceptable salts (especially hydrochloride or hydrochloride monohydrate) which is used for treating the symptoms of mild to moderate Alzheimer's disease (Sathyan et al., 1995). Molecular weight of tacrine is 198,27 and partition coefficient of tacrine (Log K) is 3,30. Tacrine (-HCl) is soluble in water and at physiological pH it is assumed to carry a positive charge. Tacrine appears to undergo extensive first-pass metabolism and is rapidly cleared from the systemic circulation. The most important pharmacokinetic parameters of tacrine are short elimination half-life $T_{1/2}$ = 1,4 - 3,6 h, Clearance CL = 150 l/h, and low peroral bioavailability (5 - 35 %). Based on these parameters, transdermal delivery of tacrine seems to be a realistic goal. After oral drug administration, the clinical tacrine concentration is about 5 - 30 ng/ml, above which unwanted side-effects are more likely to take place (Wagstaff and McTavish, 1994).

By transdermal delivery of tacrine one may: 1. minimize first-pass metabolism in GI-tract and liver; 2. provide fairly constant blood levels for extended period of time, and 3. reduce the incidence of gastrointestinal side-effects and hepatotoxicity associated with peroral tacrine administration.

In the system for iontophoretic transdermal delivery, a first device containing the pharmaceutical composition according to the invention is attached to the patient's skin, e.g. by an adhesive layer attached thereto. This device is connected to either the plus or the minus pole of the direct current source. The choice of plus or minus pole depends on whether the drug is in cation or anion form. A second device, which may or may not contain any drug component, is attached to the patient's skin at a certain distance from said first device. The delivery rate of the drug through the patient's skin is controlled by varying the voltage of the current source.

Said second device can be any electrically conductive device. According to a preferred embodiment, this device is similar to the first device except that it does not contain the drug which is added to the first device. Alternatively, this second device could contain the drug but then the drug concentration must be other than that of the first device. According to another alternative, the second device may contain a drug that is different from the drug in the first device.

The first and the second device are placed on a body part to which the drug administration is directed or to a body part suitable for transdermal administration. The two devices are placed so that the gradient of the electromotoric force is as high as possible.

The plasma concentration of the drug can be controlled by adjusting the voltage of the current source.

According to one embodiment, a salt bridge connecting a drug reservoir and skin as described in WO 93/17755 can alternatively be used as current source.

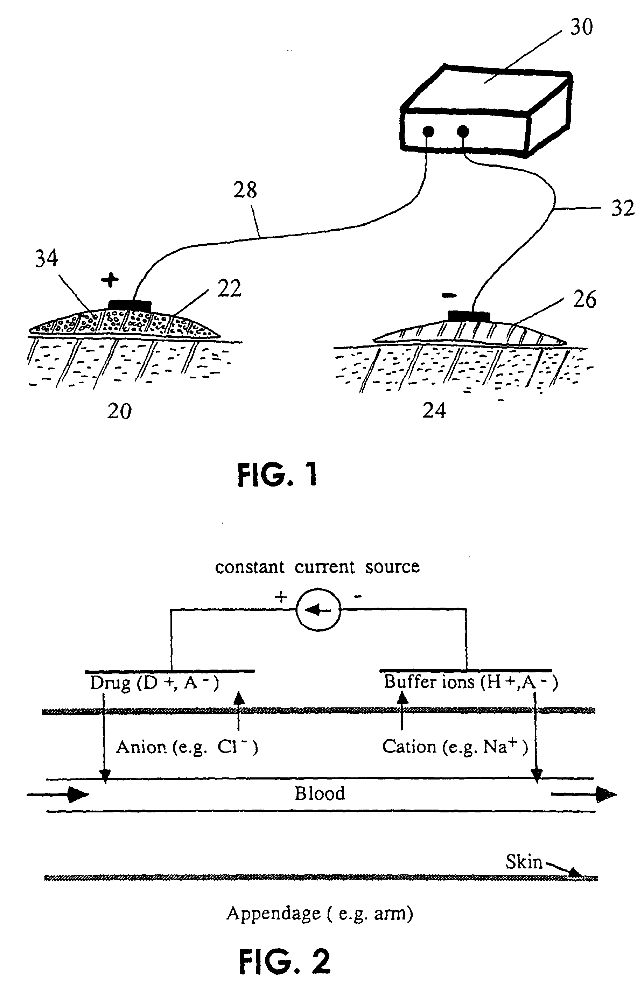

Figure 1 shows the system according to one embodiment of the invention. A first device 22 containing the drug to be administered and a second device 26 are attached to a patient's skin 20 and 24, respectively. The first device 22 is prepared from a textile fiber with a ion exchanger group attached thereto. The drug 34, which is in ionized form or which easily can be ionized, is bound to the ion exchanger group. The second device 26 is, for example, identical to the first device 22 except that said second device does not contain any drug. The devices 22 and 26 are connected to the direct current source 30 by the cables 28 and 32. The electrical current causes an effective release of the drug from the first device 22 to the patient's plasma. The desired plasma concentration of the drug is achieved by adjusting the voltage of the current source. The drug delivery can be performed continuously or intermittently.

The two devices 22, and 26 can alternatively be incorporated in one single patch to be attached to the patient's skin.

Figure 2 shows as an example the principle of iontophoretic drug delivery (Burnette R.R., 1989). The constant current iontophoretic device is attached to an appendage such as the arm. Under the electrodes exists an aqueous solution which on the anode side contains a positively charged drug ($D^+$) and its counterion ($A^-$). The drug is assumed to act as its own buffer. Under the cathode exists a buffer which is designated in its dissociated form as $H^+$ and $A^-$. Underneath the skin exists the extracellular fluid which has $Na^+$ as its primary cation and $Cl^-$ as its primary anion. It has been assumed for simplicity that only these two extracellular ions participate in iontophoretically induced charge transfer across the skin. A substantial fraction of charge can be carried by ions other than the drug, thus markedly lowering the amount of drug per unit time which can be transported via iontophoresis.

The described delivery system may be particularly suitable for iontophoretic administration of levodopa (i.e. (-)-3-(3,4-dihydroxyphenyl)-L-alanine; $C_9H_{11}NO_4$) to the patient's cerebral circulation. To the device 22 (figure 1), which is the +pole, is added levodopa. The second device 26 does not contain any levodopa. The suitable voltage is about 100 to 1000 mV.

The following experiments demonstrate the invention.

EXPERIMENTS

Example 1

1. Introduction

Physicochemical properties of drugs e.g. molecular weight and oil/water partition properties affect the drug feasibility for transdermal administration. Generally, small lipophilic and uncharged molecules permeate skin more easily than large, polar and charged ones. At physiological pH human skin carries a net negative charge (Hirvonen 1994). The negative charge results from a greater number of carboxylic acid groups over amine moieties in the proteins on the skin surface, or from specific adsorption of ions on the skin surface. Thus, skin acts like an ion-exchange membrane and as a result cations may more easily permeate the skin than anions.

The aim of this study was to evaluate the suitability of a anionic ion-exchange fiber (AIEF) for transdermal drug delivery of ionic compounds by *in vitro* tests. Sodium salicylate (mw 160.1 g/mol), pKa 3.0 and 13.4) (Gynther 1993) was used as a model drug in the test.

2. Methods

2.1 Preparation of fiber discs containing sodium saliculate

In the experiments below a woven cloth was used comprising a cotton textile fiber (RAIEX AK II) in which tertiary amine groups ($-N^+(CH_3)_3$) had been grafted to the polysaccharide molecules. The surface weight of the cloth was 200 g/m$^2$. The thickness of the fiber was 30 - 40 $\mu$m, strength 10 - 20 kg s/mm$^2$, break stretch 15 - 40 % and the ion exchanger capacity 3.3 mekv/g.

For release studies circular discs (diameter 25 mm) were cut from the above mentioned anionic ion-exchange fiber cloth. The fiber discs were washed by 5 % $NaHCO_3$ solution in a dropfunnel until all chloride was exchanged (tested by silver nitrate in $HNO_3$ solution). Thereafter the discs were treated with 5 % sodium salicylate solution. The discs containing salicylate were dried in 37 °C.

2.2 Drug release *in vitro*

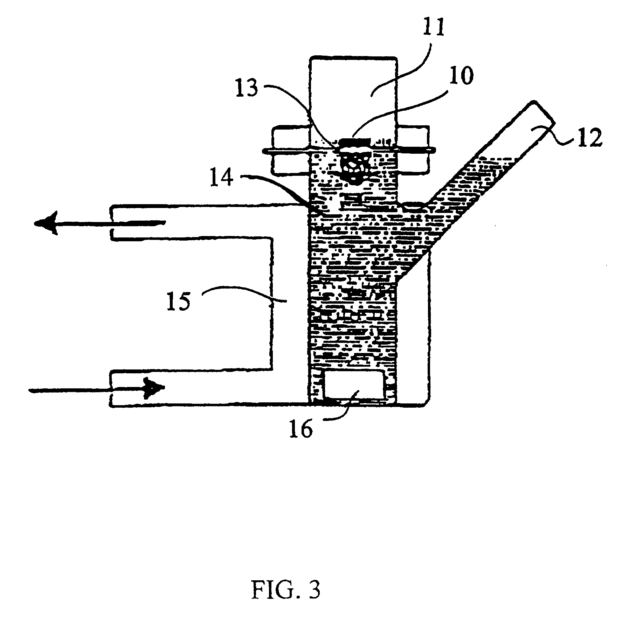

Drug release *in vitro* from the fiber discs at 37 °C was tested in Franz diffusion cells (Crown Glass Co. Inc., Somerville, NJ) (Fig. 3). In the figure reference number 11 denotes the donor compartment, 14 receiver compartment, 15 thermostat, 16 stirring rod and 12 sample tube. The fiber discs 10 were placed in the diffusion cells so that one side of the anionic ion-exchange fiber was exposed to the dissolution medium. The dissolution medium was pH 7.4 phosphate buffer saline (PBS, 143 mM, $\mu$=0.15 ($\mu$ = ionic strength)) in experiment 1 and pH 7.4 phosphate buffer (6 mM, $\mu$=0.2) in experiments 2 and 3. To assess the effect of the skin on drug release (experiment 3), a piece of epidermis 13 was placed between the fiber disc and buffer solution. At fixed times, samples of 250 $\mu$l were withdrawn and the drug concentration in the samples were determined by using HPLC (Beckman System Gold, Beckman Instrument, San Ramon, CA) with a Supelcosil LC-18-DB column (5 $\mu$m, 150 x 4.6 mm) (Supelco Inc., Rohm and Haab Co., Bellefonte, PA). The mobile phase was a binary mixture of methanol (40 % v/v) and pH 7.0 phosphate buffer (60 %). The detection wavelenght was 298 nm, and the flow rate of 1.0 ml/min, the retention time was 2.6 min.

3. Results

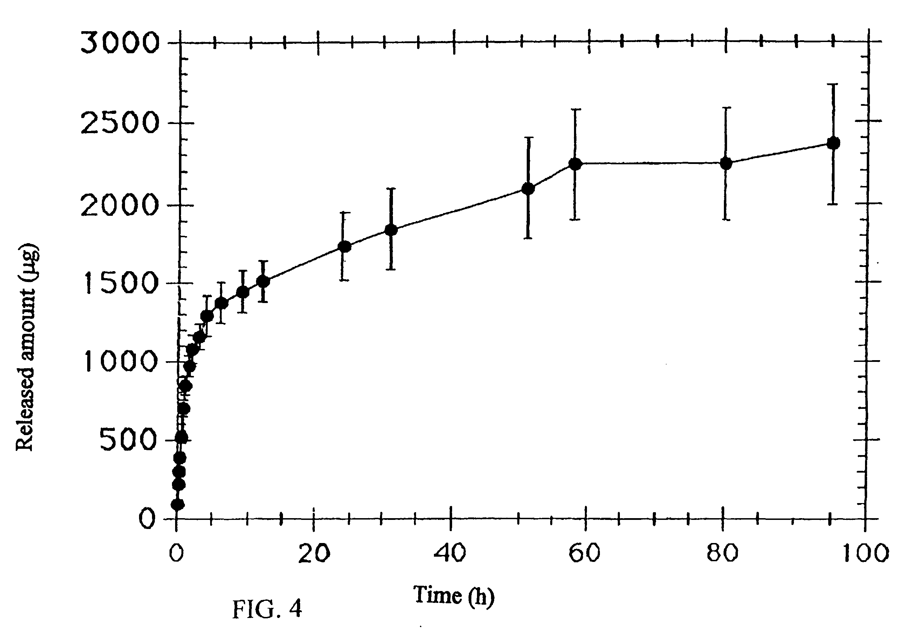

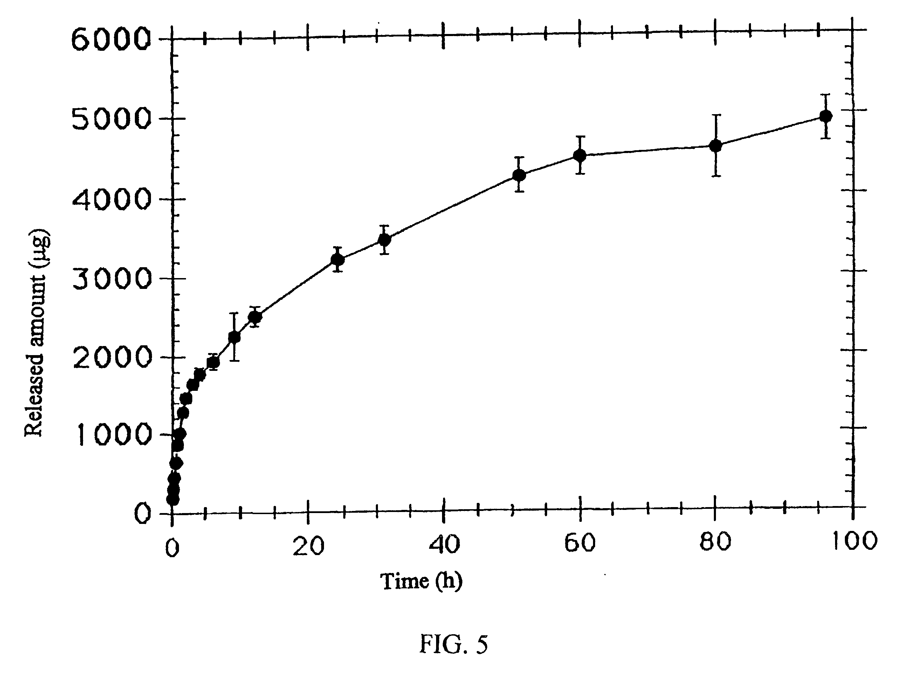

*In vitro* release of salicylate from anionic ion-exchange fiber in PBS solution (143 mM, (143 mM, $\mu$=0.15) was very rapid in the beginning of the experiment 1 (about 350 $\mu$g/h/cm$^2$) (Fig. 4). After initial burst (about < 6 h) salicylate were released from the fiber discs very slowly but at a nearly constant rate (about 20-30 $\mu$g/h/cm$^2$). Also in experiment 2 performed with phosphate buffer (6 mM, $\mu$=0.20) the burst effect was observed but during the constant release phase (about 50 h) the rate of salicylate release was higher (about 60 $\mu$g/h/cm$^2$) than in PBS solution (Fig. 5).

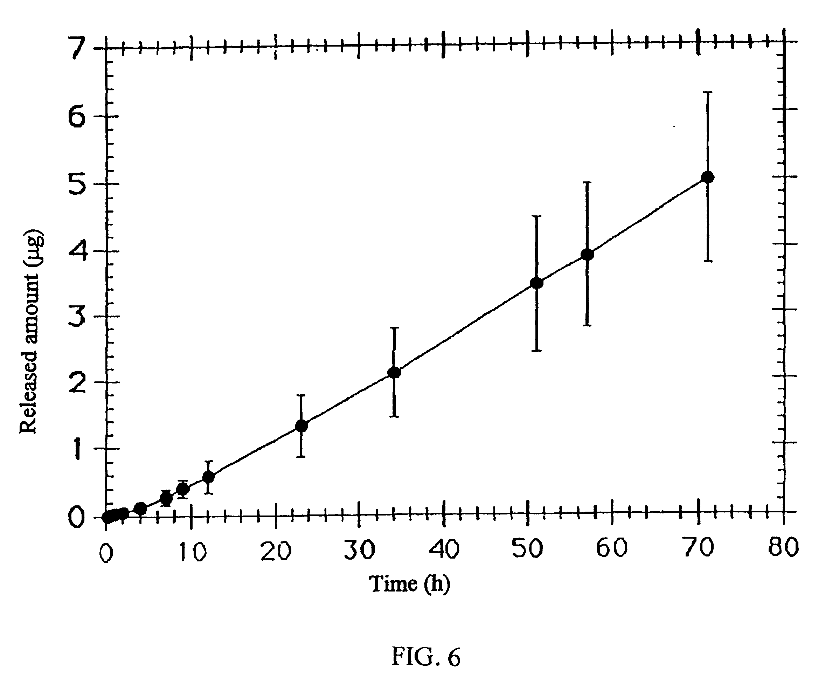

The release of salicylate from the discs was decreased markedly by effect of the skin in experiment 3. Salicylate release from anionic ion-exchange fiber was negligible (0.08 $\mu$g/h) and it followed zero-order kinetic after initial lag time (Fig. 6).

4. Discussion

The release rate and the released amount of salicylate were higher in phosphate buffer (6 mM, $\mu$=0.20) than in PBS solution (143 mM, $\mu$=0.15). This may be due to the better ion-exchange properties of phosphate buffer containing more NaCl. After initial burst, the drug was released at nearly constant rate (about 60 $\mu$g/h/cm$^2$) for two days. This shows that with the tested anionic ion-exchange fiber it seems to be possible to control drug release.

Permeation of salicylate through epidermis was substantially slower (about 0.08 $\mu$g/h) than its release directly to the buffer solution. With skin, the burst effect disappeared. Lag time before steady state release was about 2 h. Thus, skin controls the transdermal salicylate delivery from the anionic ion-exchange fiber due to the poor skin permeability of anionic salicylate. This is supported to be the observation that the *in vitro* permeability of anionic salicylate through skin was about 0.34 $\mu$g/cm$^2$/h in pH 7.0 phosphate buffer (200 mM) containing 6.27 g/l NaCl when the concentration of drug in the donor solution was 50 $\mu$g/ml (Hirvonen 1994). Nevertheless, it is possible that the *in vitro* permeation through the skin might underestimate the potential total amount of bioavailable drug (Brain et al 1993). *In vitro* the contact between the skin and the anion exchange fiber may be poorer than *in vivo*.

5. Conclusions

With the tested anionic ion-exchange fiber it is possible to control the rate of salicylate release for several days *in vitro*. However, anionic salicylate is not the best model drug for the transdermal feasibility test of ion-exchange fibers due to the cation selective properties of the human skin. Cationic drugs are more suitable for transdermal administration (e.g. clonidine) and their skin permeability is also typically higher than anionic ones. It is therefore stronly believed still better results will be obtained for cationic ion-exchange fiber in the delivery of positive charged drugs.

Example 2

Preliminary test concerning transdermal delivery of tacrine hydrochloride monohydrate

Tacrine hydrochloride monohydrate ($C_{13}H_{14}N_2$ x HCl x $H_2O$, in the following abbreviated as $TNH_3Cl$), Sigma-Aldrich GmbH, was dissolved in water to give a 4.2 % solution. An acidic fiber ion exchanger was treated with this solution, wherein the following reaction occurred:

$$TNH_3Cl + R\text{-}SO_3H \text{ -----> } TNH_3^+SO_3^-R + HCl$$

wherein R is the back bone of the fiber ion exchanger (polypropylene). The fiber ion exchanger obtained contained 30 mg of tacrine per gram of fiber ion exchanger.

The ion exchange prformance of this fiber ion exchanger was tested in a Franz diffusion cell (see Figure 3) to

- a buffert solution (pH = 7), and
- to blood through skin.

In both tests pharmacologically significant amounts of tacrine was delivered to the substrate.

Example 3

**Transdermal release, permeation and delivery of tacrine**

Methods

1. Preparation of fiber discs containing tacrine

To study tacrine release, circular discs (diameter 25 mm) were cut from the cationic ion-exchange fiber (Minsk, Belorussia). The cationic fiber discs were washed in 5 % HCl solution in a dropfunnel until all sodium was exchanged. Thereafter the discs were treated with 5 % tacrine (-HCl) solution. The discs containing tacrine were dried in 37 °C and each disc was analyzed to contain 16,7 mg of tacrine on the average. This relates to about 3,5 % (mass/mass) tacrine content in the disc.

2. Tacrine release from the ion-exchange fiber

Drug release from the fiber discs was tested *in vitro* in Franz diffusion cells (Crown Glass Co., Somerville, NJ) at 37 °C. The fiber discs were placed in the diffusion cells so that one side of the cationic ion-exchange fiber was exposed to the dissolution medium (phosphate-buffer 6 mM, pH 7,4). Surface area of the fiber discs exposed to the buffer was 0,64 $cm^2$. Samples were withdrawn up to 72 h at fixed intervals and tacrine concentration in the samples was determined by HPLC (Beckman System Gold, Beckman Instruments Inc., San Ramon, CA). The column used was Supelcosil LC-18-DB (5 m, 150 mm x 4,6 mm), and the mobile phase included 22 % of acetonitrile, 1 % of triethylamine, and 77 % of phosphate buffer at pH 6,5. Detection wavelength was 240 nm and flow rate was 1,0 ml per min.

3. Tacrine permeation across human skin *in vitro*

Transdermal permeation of tacrine across human skin *in vitro* was studied with Side-by-Side -diffusion chambers (DC-100, Crown Glass Co., Somerville, NJ) at room temperature (ca. 25 °C). Donor phase (3 ml) contained: 1,5 % solution of tacrine (-HCl) in HEPES-buffer (pH 7,4), and 2. cationic ion exchange fiber with 3,5 % of tacrine in HEPES-buffer. Samples were withdrawn up to 120 h at fixed intervals and tacrine concentration in the samples was determined by HPLC. Transdermal flux of tacrine (g/h per $cm^2$) across the skin was calculated using linear regression of the straight-line portion of drug permeation vs. time curve, and dividing by the surface area of the skin (0,64 $cm^2$).

4. Iontophoretic regulation of tacrine permeation

Electrodes for iontophoresis were prepared from silver wire and silver chloride (Aldrich-Chemie, Steinheim, Germany). Direct current (0,5 mA/cm$^2$) during iontophoresis was delivered by HP 6181C DC current source (Hewlett-Packard, Cupertino, CA) from the electrodes to the diffusion chambers via salt bridges. Salt bridges were prepared by injecting 1 M NaCl-gel (3 % agar) inside plastic tubing (diameter 4 mm, length ca. 15 cm). Salt bridges prevented direct contact and possible reactions of tacrine with Ag/AgCl-electrodes. Transdermal permeation of ions increase most during iontophoresis due to electrostatic repulsion. Therefore, experiments were performed at pH 7,4, when tacrine is mainly in ionized form. Because Ag/AgCl-electrodes cause precipitation of phosphates, we used HEPES-buffer at pH 7,4 during the iontophoresis experiments. AgCl-cathode was connected via the salt bridge to the donor solution that included the negatively charged tacrine (5 % solution, 3,5 % in cationic ion exchange fiber, 0,05 % in activated charcoal); salt bridge connected also the receiver solution to the positive silver anode. The chambers were connected in series as constant DC-current was used. The Ag/AgCl-electrodes prepared could be used continuously 12 h, whereafter passive permeation of tacrine was followed up to 72 h.

Results

1. Fiber discs containing tacrine

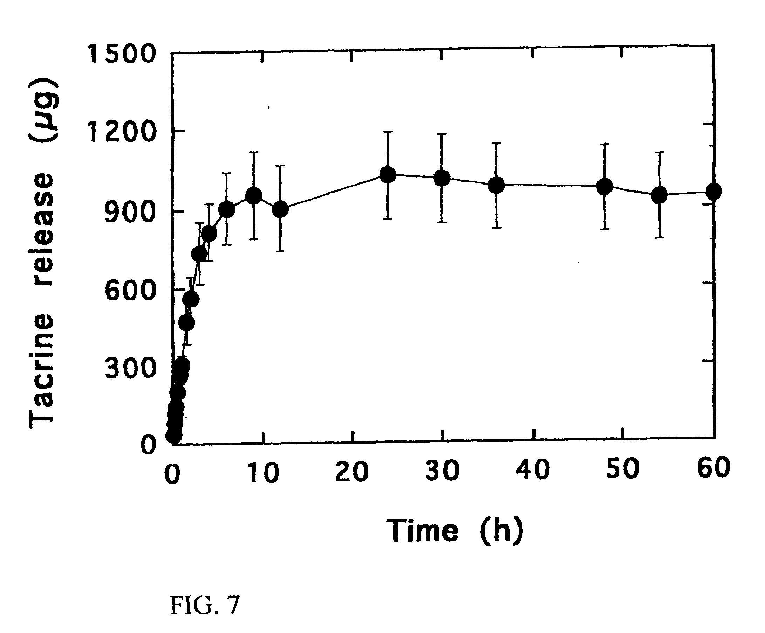

Positively charged tacrine reacts chemically with the negative groups in the cationic ion exchange fiber. However, release of tacrine from the cationic ion-exchange fiber was very rapid in the beginning of the experiment (Fig. 7). After initial burst of about 6 - 12 h, 50 % of tacrine was released from the fiber, whereafter further drug release was prevented by the reached equilibrium of tacrine in the buffer and the ion-exchange fiber. Tacrine release rate from the cationic ion-exchange fiber during the first 10 hours was about 375 g/h per cm$^2$. Therefore, cationic ion-exchange fiber seems very suitable material for a drug reservoir that can release tacrine in a controlled fashion.

2. Tacrine permeation across human skin *in vitro*

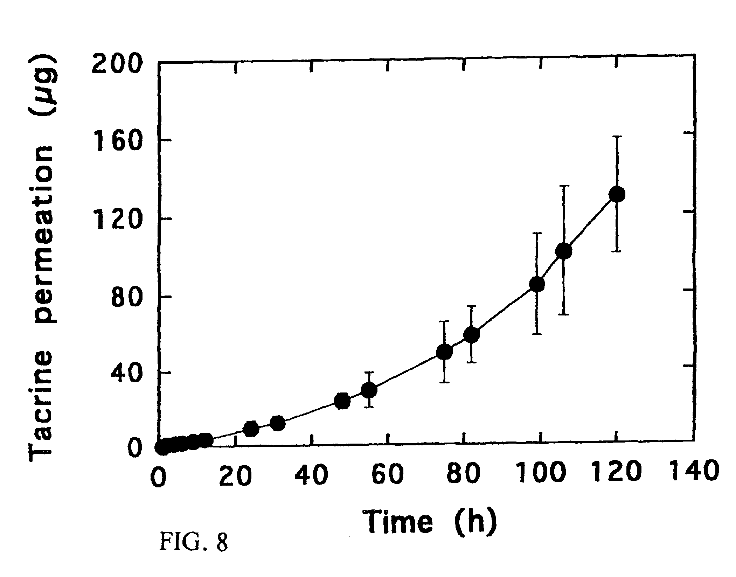

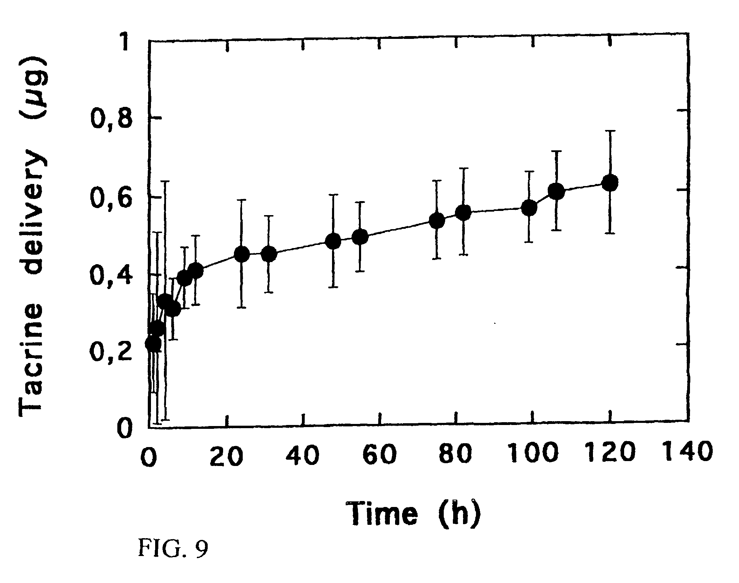

Human skin carries a net negative charge at neutral and basic pH (Burnette, 1989) and, therefore, the skin may "enhance" the permeation of positive tacrine. Transdermal permeation of tacrine across human skin at pH 7,4 is presented in Fig. 8. Steady-state flux of tacrine (5 % - solution) across human skin *in vitro* was 2,95 g/h per cm$^2$. Lag-time of permeation was long, ca. 24 h, until tacrine had penetrated through the skin into the receiver phase (Fig. 8). Delivery of tacrine across the skin from the cationic ion-exchange fiber was very low, about 0,003 g/h per cm$^2$ (Fig. 9). Drug delivery from the fiber was constant for 120 h, but the permeation rate of tacrine was about three orders of magnitude lower than in the case of tacrine solution. This implies that tacrine "prefers" the ion-exchange fiber over the skin as a permeation "target".

3. Transdermal iontophoresis

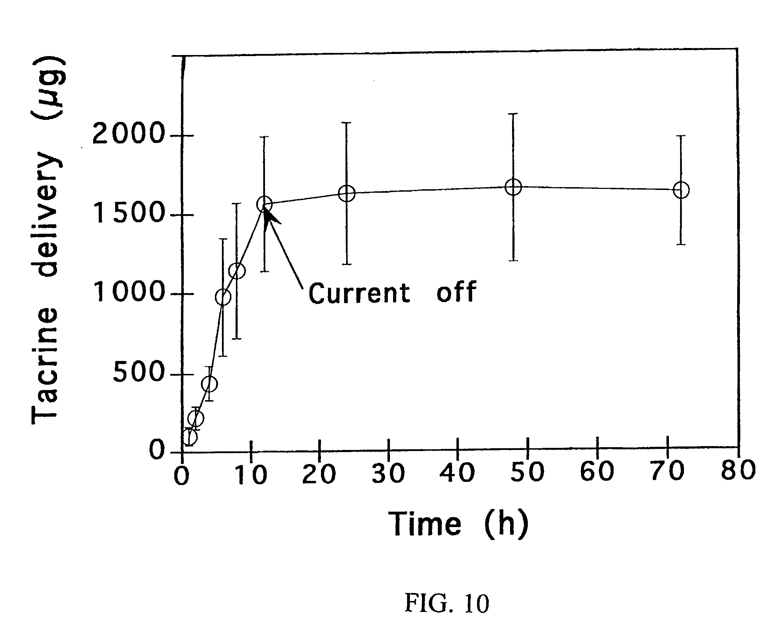

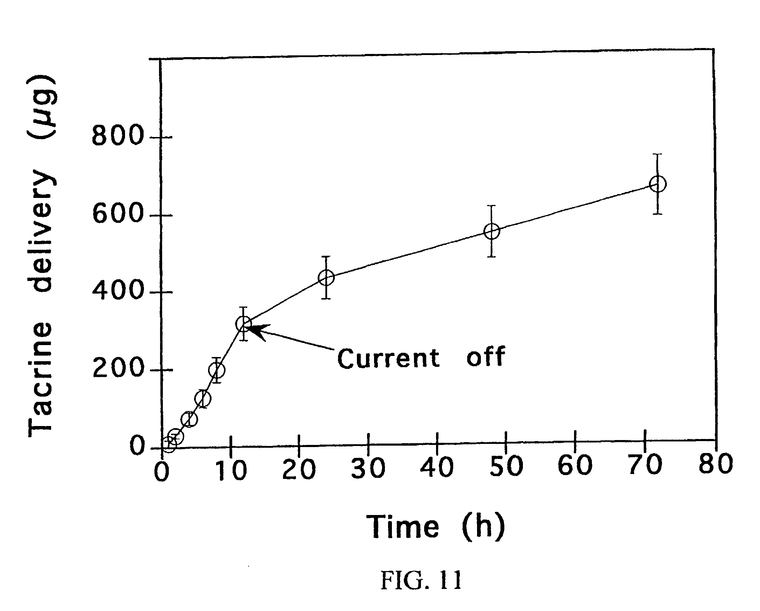

Transdermal permeation of charged drugs can be increased with small electric current, iontophoresis (Burnette, 1989). As a weak base tacrine is positively charged below it's pKa-value, *i.e.*, at neutral and acidic pH. Therefore, tacrine can be delivered from the anode (positive electrode), as electrostatic repulsion "forces" the ion with the same charge across the skin. Penetration rate of drugs during transdermal iontophoresis can be regulated by the current used. In practice, the upper limit for current is about 0,5 mA/cm$^2$, above which skin reactions, mostly redness, are typical (Ledger, 1992).
Iontophoresis was clearly an effective method to increase transdermal permeation of tacrine. Steady-state flux of tacrine at pH 7,4 during constant current iontophoresis was 218 µg/ h per cm$^2$. Iontophoresis increased tacrine permeation almost 100-fold compared to passive permeation (Table 1). In addition, lag-time of drug permeation was reduced to about 0,5 h, whereafter penetration rate of tacrine remained constant until the current was switched off (Fig. 10). After the current termination transdermal flux of tacrine returned to the passive level rapidly. This indicates that iontophoresis has a reversible action also *in vitro* and the skin returns to impermeable membrane when the current is terminated.
Iontophoretic delivery of tacrine across the skin from the cationic ion-exchange fiber, 44,1 µg/ h per cm$^2$, was ower 10 000 -fold compared to passive permeation from the ion-exchange fiber (Table 1). Fig. 11 clearly illustrates, that iontophoretic delivery of tacrine is constant as long as the current is on. This result clearly indicates that electrical triggering can be utilized in controlling tacrine release from the cationic ion-exchange fiber reservoir for transdermal drug delivery purposes.

4. Transdermal delivery of tacrin for systemic use

Based on the physical-chemical properties, tacrine is a promising candidate for transdermal delivery. Tacrine clearance from the body is about 150 l/h and the maximal concentration of tacrine in the plasma after oral absorption is small, i.e. about 5 - 30 ng/ml (Wagstaff and McTavish, 1994). Expected steady-state drug concentration in the plasma ($c_{ss}$, ng/ml) during transdermal drug delivery can be calculated as

$$c_{ss} = A k_0 / CL$$

where A = absorption area on the skin surface ($cm^2$), $k_0$ = drug flux across the skin ($\mu g/h$ per $cm^2$), and CL = clearance (l/h) (Guy and Hadgraft, 1989). Maximum size of one transdermal delivery system is about 100 $cm^2$, but more than one system can be used simultaneously, if needed.

Based on the previous parameters the calculated steady-state plasma concentrations of tacrine (ng/ml) are presented in Table 1 (100 $cm^2$ system). Passive delivery of tacrine (5 % solution) is expected to achieve steady-state plasma concentrations of about 2 ng/ml. From the cationic ion-exchange fiber passive delivery of tacrine reaches a much lower plasma concentration of about 0,002 ng/ml (Table 1). Thus, transdermal delivery of tacrine seems possible, but its permeation across the skin should preferably be improved. The experiments above show that transdermal permeation of tacrine and charged drugs in general can be increased with a small electric current, iontophoresis.

Tacrine permeation from 5 % solution achieves plasma concentrations over 200 ng/ml, which means that current density and/or the size of the transdermal patch can be reduced considerably to achieve clinically acceptable tacrine concentration (5 - 30 ng/ml). Combination of iontophoresis and cationic ion-exchange fiber provides also adequate delivery across the skin (about 30 ng/ml, Table 1).

It will be appreciated that the compositions of the present invention can be incorporated in the form of a variety of embodiments, only a few of which are disclosed herein. It will be apparent for the specialist in the field that other embodiments exist and do not depart from the spirit of the invention. Thus, the described embodiments are illustrative and should not be construed as restrictive.

Table 1

| Tacrine premeation across human skin *in vitro.* Average ± standard deviation, N= 4-7. Expected steady-state plasma concentration of tacrine ($c_{ss}$, ng/ml) was calculated using the equation $c_{ss} = A k_0 / CL$, where A = surface area for drug adsorption (100 $cm^2$), $k_0$ = steady-state flux of tacrine across the skin ($\mu g/h$ per $cm^2$), and CL = clearance (150 l/h). Direct current iontophoresis (0.5 $mA/cm^2$) was on for 12 h. | | |
|---|---|---|
| Test (Tacrine content) | Tacrine permeation | |
| | Flux ($\mu g/$ h per $cm^2$) | $c_{ss}$ (ng/ml) |
| Passive permeation (5 % solution) | 2.95±0.66 | 1.97 |
| Passive permeation (3.5 % in ion-exchange fiber) | 0.003±0.0006 | 0.002 |
| Iontophoretic permeation (5 % solution) | 218±46.2 | 145 |
| Iontophoretic permeation (3.5 % in ion exchange fiber) | 44.1±6.73 | 29.4 |

References

Brain KR, Hadgraft J. James VJ, Shah VP, Walters KA, Watkinson AC: *In vitro* assessment of skin permeation from a transdermal system for the delivery of oestradiol. Int J Pharm 89:R13-R16, 1993

Burnette R.R.: Iontophoresis In Transdermal Drug Delivery, pp. 247-292, Eds. Guy R.H. and Hadgraft J., Marcel Dekker Inc., New York and Basel, 1989

Guy RH, Hadgraft J.: Transdermal drug delivery: a perspective. J Contr Rel 4: 237-251, 1987

Guy RH, Hadgraft J: Selection of drug candidates for transdermal drug delivery. In Transdermal Drug Delivery. pp. 59-81. Eds. Hadgraft J, Guy RH, Marcel Dekker Inc., New York, 1989

Gynther J: Lääkeainekemian perusteet. Fortis, Graafiset palvelut, Kuopio, 1993

Hirvonen J: Enhancement of transdermal drug penetration with dodecyl N, N-dimethylamino acetate and iontophoresis. Academic Dissertation, University of Kuopio, 1994

Ledger P.W.: Skin biological issues in electrically enhanced transdermal delivery, Adv. Drug. Deliv. Rev., 9: 289-307, 1992

Sathyan G et al., Transdermal delivery of tacrine: I. Identification of a suitable delivery vehicle. Int.J.Pharm., 114:75-83, 1995

Wagstaff A J and McTavish D: Tacrine: A review of its pharmacodynamic and pharmacokinetic properties, and therapeutic efficacy in Alzheimer's disease. Drugs and Aging, 4: 510-540, 1994.

## Claims

1. A pharmaceutical composition for the controlled iontophoretic transdermal delivery of a drug comprising a combination of a drug and an electrically conductive carrier, wherein the administration of the drug to the patient is intended to be enhanced by leading an electrical current from said composition to the patient's skin, **characterized in that** the electrically conductive carrier is a textil fiber with a ion exchanger group grafted thereto.

2. The pharmaceutical composition according to claim 1 **characterized in that** the textile fiber is selected from the group consisting of wool, cotton, flax fibers and fibers of cellulose or its derivatives, polyethylene, polypropylene, polystyrene, polyamide fibers and carbon fibers.

3. The pharmaceutical composition according to claim 1 or 2 **characterized in that** a cation has been grafted to the fiber.

4. The pharmaceutical composition according to claim 1 or 2 **characterized in that** an anion has been grafted to the fiber.

5. The pharmaceutical composition according to claim 3 **characterized in that** the fiber is a cotton fiber to which the tertiary amine cation $-N^+(CH_3)_3$ has been grafted.

6. The pharmaceutical composition according to any one of the claims 1 - 5 **characterized in that** the textile fiber is a woven cloth.

7. The pharmaceutical composition according to any one of the previous claims **characterized in that** the drug is tacrine or its pharmaceutically acceptable salt.

8. A system for the iontophoretic transdermal delivery of a drug to a patient, wherein said system comprises

   - a first device (22) comprising the drug to be administered,
   - a second device (26), wherein said devices (22, 26) are intended to be attached to the patient's skin,
   - a source (30) of direct current, and
   - means (28, 32) for connecting said current source (30) to said first device (22) and to said second device (26), **characterized in that** said first device is a pharmaceutical composition according to any of the claims 1 - 7.

9. The system according to claim 8, **characterized in that** said second device (26) does not contain any drug.

10. The system according to claim 8 or 9, **characterized in that** said first device (22) and said second device (26) are pads having adhesive layers to be attached to the patient's skin.

## Patentansprüche

1. Pharmazeutische Zusammensetzung zur kontrollierten iontophoretischen transdermalen Abgabe eines Medikaments, umfassend eine Kombination aus einem Medikament und einem elektrisch leitenden Träger, wobei die Verabreichung des Medikaments an den Patienten durch Leiten eines elektrischen Stroms von der Zusammensetzung zu der Haut des Patienten verstärkt werden soll, **dadurch gekennzeichnet, dass** der elektrisch leitende Träger eine Textilfaser mit einer darauf gepfropften Ionenaustauschergruppe ist.

2. Pharmazeutische Zusammensetzung nach Anspruch 1, **dadurch gekennzeichnet, dass** die Textilfaser aus der Gruppe, bestehend aus Woll-, Baumwoll-, Flachsfasern und Fasern aus Zellulose oder ihren Derivaten, Polyethylen-, Polypropylen-, Polystyrol-, Polyamidfasern und Kohlenstofffasern ausgewählt ist.

3. Pharmazeutische Zusammensetzung nach Anspruch 1 oder 2, **dadurch gekennzeichnet, dass** ein Kation auf die Faser aufgepfropft wurde.

**4.** Pharmazeutische Zusammensetzung nach Anspruch 1 oder 2, **dadurch gekennzeichnet, dass** ein Anion auf die Faser aufgepfropft wurde.

**5.** Pharmazeutische Zusammensetzung nach Anspruch 3, **dadurch gekennzeichnet, dass** die Faser eine Baumwollfaser ist, auf die das tertiäre Amin-Kation $-N^+(CH_3)_3$ aufgepfropft wurde.

**6.** Pharmazeutische Zusammensetzung nach einem der Ansprüche 1 bis 5, **dadurch gekennzeichnet, dass** die Textilfaser ein Gewebe ist.

**7.** Pharmazeutische Zusammensetzung nach einem der vorangehenden Ansprüche, **dadurch gekennzeichnet, dass** das Medikament Tacrin oder sein pharmazeutisch annehmbares Salz ist.

**8.** System für die iontophoretische transdermale Abgabe eines Medikaments an einen Patienten, wobei das System umfasst:

- eine erste Vorrichtung (22), die das zu verabreichende Medikament umfasst,
- eine zweite Vorrichtung (26), wobei die Vorrichtungen (22, 26) dazu bestimmt sind, um an der Haut des Patienten befestigt zu werden,
- eine Quelle (30) für Gleichstrom
- Mittel (28, 32) zum Anschließen der Stromquelle (30) an die erste Vorrichtung (22) und die zweite Vorrichtung (26), **dadurch gekennzeichnet, dass** die erste Vorrichtung eine pharmazeutische Zusammensetzung nach einem der Ansprüche 1 bis 7 ist.

**9.** System nach Anspruch 8, **dadurch gekennzeichnet, dass** die zweite Vorrichtung (26) kein Medikament enthält.

**10.** System nach Anspruch 8 oder 9, **dadurch gekennzeichnet, dass** die erste Vorrichtung (22) und die zweite Vorrichtung (26) Pads sind, die zur Befestigung an der Haut des Patienten Klebstoffschichten haben.

**Revendications**

**1.** Composition pharmaceutique pour la délivrance transdermique ionophorétique maîtrisée d'un médicament comprenant une combinaison d'un médicament et d'un véhicule électriquement conducteur, dans laquelle l'administration du médicament au patient est prévue pour être favorisée en conduisant un courant électrique à partir de ladite composition vers la peau du patient, **caractérisée en ce que** le véhicule électriquement conducteur est une fibre textile à laquelle est greffé un groupe échangeur d'ions.

**2.** Composition pharmaceutique selon la revendication 1, **caractérisée en ce que** la fibre textile est choisie dans le groupe formé par la laine, le coton, les fibres de lin et les fibres de cellulose ou ses dérivés, le polyéthylène, le polypropylène, le polystyrène, les fibres polyamides et les fibres de carbone.

**3.** Composition pharmaceutique selon la revendication 1 ou 2, **caractérisée en ce qu'**un cation a été greffé à la fibre.

**4.** Composition pharmaceutique selon la revendication 1 ou 2, **caractérisée en ce qu'**un anion a été greffé à la fibre.

**5.** Composition pharmaceutique selon la revendication 3, **caractérisée en ce que** la fibre est une fibre de coton à laquelle le cation amine tertiaire $-N^+(CH_3)_3$ a été greffé.

**6.** Composition pharmaceutique selon l'une quelconque des revendications 1 à 5, **caractérisée en ce que** la fibre textile est un tissu tissé.

**7.** Composition pharmaceutique selon l'une quelconque des revendications précédentes, **caractérisée en ce que** le médicament est la tacrine ou un de ses sels pharmaceutiquement acceptables.

**8.** Système pour la délivrance transdermique ionophorétique d'un médicament à un patient, dans lequel ledit système comprend :

- un premier dispositif (22) comprenant le médicament à administrer ;

- un deuxième dispositif (26), dans lequel lesdits dispositifs (22, 26) sont prévus pour être fixés à la peau d'un patient ;
- une source (30) de courant continu ; et
- des moyens (28, 32) pour connecter ladite source de courant (30) audit premier dispositif (22) et audit deuxième dispositif (26), **caractérisé en ce que** ledit premier dispositif est une composition pharmaceutique selon l'une quelconque des revendications 1 à 7.

9. Système selon la revendication 8, **caractérisé en ce que** ledit deuxième dispositif (26) ne contient aucun médicament.

10. Système selon la revendication 8 ou 9, **caractérisé en ce que** ledit premier dispositif (22) et ledit deuxième dispositif (26) sont des tampons dont les couches adhésives sont destinées à être fixées à la peau d'un patient.

**FIG. 1**

constant current source

Drug (D +, A⁻)  Buffer ions (H +, A⁻)

Anion (e.g. Cl⁻)  Cation (e.g. Na⁺)

Blood

Skin

Appendage ( e.g. arm)

**FIG. 2**

FIG. 3

FIG. 4

EP 0 904 128 B1

FIG. 5

FIG. 6

FIG. 7

EP 0 904 128 B1

FIG. 8

FIG. 9

EP 0 904 128 B1

FIG. 10

EP 0 904 128 B1

FIG. 11